Europäisches Patentamt

European Patent Office

Office européen des brevets

(11) Publication number: **0 092 383**
**B1**

(12) # EUROPEAN PATENT SPECIFICATION

(45) Date of publication of patent specification: **25.11.87**

(51) Int. Cl.⁴: **A 61 B 17/08**, A 61 L 17/00

(21) Application number: **83302105.8**

(22) Date of filing: **14.04.83**

(54) Silicone coated surgical staple.

(30) Priority: **15.04.82 US 368492**

(43) Date of publication of application:
**26.10.83 Bulletin 83/43**

(45) Publication of the grant of the patent:
**25.11.87 Bulletin 87/48**

(84) Designated Contracting States:
**AT BE CH DE FR GB IT LI NL SE**

(56) References cited:
**FR-A-2 072 066**
**FR-A-2 254 352**
**GB-A-2 069 650**
**US-A-4 140 125**

(73) Proprietor: **ETHICON INC.**
**U.S. Route 22**
**Somerville New Jersey 08876 (US)**

(72) Inventor: **Gertzman, Arthur Albert**
**785 Partridge Drive**
**Bridgewater New Jersey (US)**
Inventor: **Zett, Mary Lou**
**24 Hillcrest Road**
**Warren New Jersey (US)**
Inventor: **Conti, James Turner**
**4 Connet Lane**
**Readington New Jersey (US)**
Inventor: **Gaterud, Mark Turner**
**RD 1, Box 183 Pinehill Road**
**Annandale New Jersey (US)**

(74) Representative: **Jones, Alan John et al**
**CARPMAELS & RANSFORD 43 Bloomsbury Square**
**London, WC1A 2RA (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

# 0 092 383

**Description**

The invention relates to an adhered array of sterile surgical staples coated with a polymeric silicone material.

Surgical stapling instruments which are actuated in a repetitive manner to discharge and form a series of surgical staples are well known. Such stapling instruments have found considerable acceptance in the closing of wounds to the skins of humans and animals. Such instruments may either be disposable or may be used repeatedly by placing a new cartridge in the stapling instrument. The staples themselves are metal staples and are made from stainless steel, tantalum, or other biologically acceptable metal. In many instances, the staples are coated with either a soap composition or, in some instances, a polytetrafluoroethylene coating to assist in both the placement of the staple and the function of the stapling instrument.

The use of silicone in medical devices has been known for some time. One use of silicone has been as a coating on catheters. Silicone has been used to reduce tissue irritation and prevent tissue ingrowth. Such catheters are more fully described in US—A—3,434,869 and US—A—3,783,454. Silicones have also been widely used in various prosthetic devices primarily because silicone is not susceptible to tissue ingrowth and generally the silicone is used in combination with other materials to develop a suitable prosthesis. Examples of such prosthesis which incorporate silicone materials are given in US—A—3,681,786, US—A—3,739,403, and US—A—3,996,623.

Silicone polymers have also been used in other medical devices because of silicone's biological compatability, minimal tissue ingrowth property, and its other physical properties as are well known. Examples of such medical devices are reinforcing elements for muscles as described in US—A—3,464,615, an instrument as described in US—A—3,962,519 and surgical tapes as described in US—A—4,140,125. FR—A—2,072,066 discloses the use of a silicone material to coat a surgical suture to facilitate entry and removal thereof during a surgical procedure.

While silicone polymers and elastomeric silicones are well known for use in medical and surgical devices, to the best of our knowledge, no one has utilized silicone on sterile surgical staples. The coatings used to improve the properties of surgical staples have been either soap compositions or polytetrafluoroethylene compositions, i.e. Teflon®. Both coatings have some disadvantages. Teflon® is degraded by gamma radiation sterilization and the lubricity of the coated product tends to vary considerably. Soap is an irritant in many environments and is humidity sensitive. It is believed it has been thought the silicones would not perform as well as other coatings as the silicone materials might "gum" or stick in the automated instruments used to set the staples and, hence, hinder the setting of the staples. Also, silicones must be carefully cured in order to achieve optimal results.

We have found that a sterile surgical staple comprising a pair of legs connected at one end by a crown with substantially the entire surface of the staple coated with a polymeric silicone material is much easier to place or set to close a wound in human or animal skin using an automatic type stapling instrument. Such a coated staple causes less trauma when being set and is easier for the surgeon to use. Furthermore, such a staple is easier to remove from the skin once the wound has healed. Either cured or uncured silicone materials may be used to coat the staples and provide the improvement in both penetration and extraction forces.

We have also discovered that the silicone may be used to adhere staples together in an array of staples. Unexpectedly, adhering the staples together with silicone does not interfere with the improvement of properties of the coated staple.

Therefore, the present invention provides an array of sterile surgical staples as defined in claim 1 and a method of producing such an array as defined in claim 5.

The invention will be more fully described in conjunction with the accompanying drawings.

Figure 1 is a perspective view of one form of sterile surgical staple;

Figure 2 is a perspective view of yet another form of a sterile surgical staple;

Figure 3 is a perspective view of an array of surgical staples according to the present invention;

Figure 4 is a cross-sectional view of an automatic stapling instrument showing an array of sterile surgical staples of the present invention loaded in said instrument;

Figure 5 is a perspective view of a wound closed using sterile surgical staples;

Figure 6a is a cross-sectional view of a wound closed with a sterile surgical staple; and

Figure 6b is a cross-sectional view of a wound depicting the sterile surgical staple being removed from the healed wound.

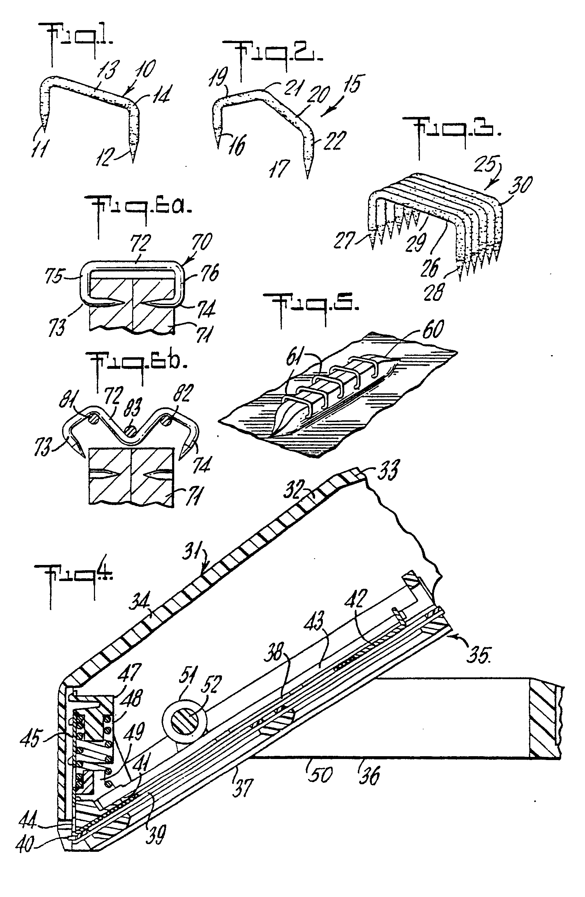

In Figure 1 there is shown one configuration of a sterile surgical staple 10. The staple comprises a pair of legs 11 and 12 connected at one end by a crown 13. In this embodiment, the crown is substantially perpendicular to the legs. Substantially all of the surface of the legs and crown is coated with a silicone elastomeric material 14. In Figure 2, the staple 15, comprises a pair of legs 16 and 17 joined at one end by a crown; however, in this configuration the crown comprises two sections 19 and 20 which extend at right angles from the legs and are connected at the apex 21. Again, substantially the entire surface of the legs and crown is coated with a silicone elastomeric material.

The thickness of the silicone coating is not critical as long as the coating covers substantially the entire surface of the staple and is reasonably uniform. It is preferred the elastomeric coating be primarily

2

composed of dimethylpolysiloxane plus a silica filler but other non-toxic silicones and fillers are also operative. For example, ethylmethypolysiloxane, 3,3,3-trifluoropropylmethylpolysiloxane and copolymers . of the above and dimethylpolysiloxane may be used. Silicone elastomeric materials are also well known and commercially sold and a detailed discussion of their composition and variations is unnecessary. Silicone elastomeric stocks which are especially prepared for medical usage are available from the Dow Corning Corporation of Midland, Michigan.

As previously discussed, the silicone coated staples are produced by initially forming the staple in its desired configuration as is well known in the art. The staple once formed is cleaned and this may be accomplished by various techniques though ultrasonic cleaning is one of the better techniques for insuring that the surface of the staples is clean.

The clean staple is coated by placing it in a bath of silicone in a suitable carrying material such as xylol or heptane. The coated staples are dried at temperatures of from room temperature to 350°C for periods of time of from 15 seconds to 5 hours to produce the coated staple. Silicone coated staples are either placed in a cartridge or placed in a suitable instrument and the cartridge or instrument placed in a package and the package sterilized using steam, dry heat, ethylene oxide or cobalt 60 isotope irradiation electron beam irradiation as is well known in the art. Silicone coatings withstand and may be sterilized by any of the well known techniques; that is dry or wet heat, ethylene oxide, electron or gamma irradiation, and other sterilization techniques well known in the art.

In Figure 3 there is shown an array 25 of staples according to the invention. Each staple 26 comprises a pair of legs 27 and 28 connected at one end by a suitable crown 29 with the surface of the legs and crown coated with a polymeric silicone coating 30. The staples are stacked or butted side to side in a suitable array. The staples are adhered together by the silicone coating so that the array acts as a unit for ready insertion into a cartridge or the working face of a stapling instrument. When using the silicone to adhere a plurality of staples together to form an array of staples, the solution from which the silicone is applied should contain 6% or more by weight of silicone solids and preferably from 9% to 12% by weight of silicone solids. To adhere the staples together the silicone needs to be cured. Suitable curing conditions are temperatures of 200°C or higher with cure times of 30 minutes or more. Preferably the curing conditions are temperatures of 200 to 300°C and times from 30 to 60 minutes.

In Figure 4 there is shown an automatic disposable skin stapling instrument. The instrument is loaded with an array of silicone coated sterile surgical staples of the present invention. In Figure 4 only the primary components of the surgical stapling instrument are shown. The stapling instrument 31 generally comprises a body 32 having a rear portion 33 which serves as the handle and a forward portion 34. The forward portion 34 of the instrument includes a surgical staple magazine indicated at 35. The instrument is actuated by the trigger mechanism 36. The magazine 35 is affixed to the lower edge of the forward portion of the body. The magazine comprises a lower member 37 and an upper member 38 with an anvil plate 39 located between these members. The anvil plate 39 terminates at its forward end in a coextensive anvil surface 40. Slidably mounted on the plate is a row of siliconized staples 41 of the present invention. Also slidably mounted on the anvil plate 39 is a feeder shoe 42 which is constantly urged towards the anvil surface 40 by the double coil spring 43. The forward end of the magazine 35 provides a channel 44 for a staple driver 45 mounted within the forward portion of the instrument body. The staple driver 45 is affixed to a staple driver actuator 47. The trigger is operatively connected to the staple driver actuator to shift it and the staple driver 45. A return spring 48 is located within the forward portion of the instrument body.

The upper end of the return spring is in contact with the staple driver actuator 47 and the lower end of the return spring is mounted on a return spring seat 49. The return spring is intended to bias the staple driver to its retracted position and at the same time to bias the trigger 36 to its normal position. The forward portion 50 of the trigger joins and is integral with a pivot pin 51. A portion of the pivot pin is adapted to be received in the side walls of the instrument body. The central portion of the pivot pin is of a reduced diameter as at 52. It provides clearance for the feeder shoe. Hence, the trigger 6 is pivotally fixed to the instrument body 32 and is operatively connected to the staple driver actuator 47 and the staple driver 45. Instruments of this type are more fully described in US—A—4,109,844 and US—A—4,179,057 assigned to Senco Products Inc. In operation of the stapling instrument, the trigger 36 is pressed and a staple 41 is formed about the anvil end 40 and inserted in the skin to close a wound. Once the staple is formed about the anvil 40 the trigger 36 is released and the instrument may be removed from the staple leaving the staple closing the wound.

To produce an adhered array of staples according to the present invention that may be used with such a stapling instrument the staples are placed on a suitable carrier or holder in abutting relationship and at the angle at which they will be disposed when placed in the stapling instrument. The array is immersed in a silicone solution or sprayed with a silicone solution or otherwise treated to coat the array with silicone. The treated array is dried and the dried array cured at temperatures of 200°C or higher for periods of time of 30 minutes or more. The array may then be loaded in the instrument and staples placed using the instrument. The adhered staples are easy to place i.e. they have decreased penetration forces as compared to untreated staples or staples treated with the soap solutions of the prior art. Also, the placed staples are easier to remove from the skin once the wound is healed, i.e. they have reduced extraction forces as compared to untreated staples or staples treated with the prior art soap solutions.

In Figure 5 there is a perspective view of a skin wound closure utilizing the staples described above.

The incised section of the wound (60) is approximated in an everted orientation and the staples 61 formed along the length of the wound to close the wound.

Figure 6a shows a staple 70 joining two sections of skin tissue 71 together. The staple is in its formed position. The staple has been bent at points along the crown 72 spaced from the legs 73 and 74 to form perpendicular areas 75 and 76 to allow the legs to be inserted into the skin. Once the wound is healed it is a relatively simple matter to remove the staple with an appropriate instrument. Such removal is schematically depicted in cross section in Figure 6b. The extractor comprises a pair of parallel stationary prongs 81 and 82. These prongs are adapted so they will fit beneath the crown 72 of the staple at its bent points. The extractor also includes a movable central lever 83 which may be pushed between the stationary prongs. The prongs are placed beneath the crown and the central lever urged between the prongs or the prongs moved upwardly about the center lever. The staple shown in Figure 6b has been extracted from the wound.

The following examples describe methods for coating staples with silicone for use in forming an array in accordance with the present invention. Example 1 also describes a prior art technique for coating staples with soap.

Example 1

A plurality of stainless steel staples are coated with silicone using a solution having the following composition:

| Syl-Off* 23—30 | Percent by weight |
|---|---|
| (30% by weight silicone in xylene) | 9.9 |
| n-Heptane | 88.8 |
| Xylol | 1.3 |

The ingredients are placed in a stainless steel container, the container covered and the solution agitated for approximately 16 hours until the solution is clear and uniform. (Syl-Off* is a trademark for a paper coating grade silicone sold by Dow Corning Corp.). The solution contains approximately 3.3 percent by weight of silicone solids. Some of the silicone solution is placed in a dish or container. Degreased stainless steel staples are placed in a sieve and the sieve lowered into the silicone solution. The staples are gently agitated for approximately 3 seconds to insure complete coverage. The sieve is lifted from the solution and the treated staples allowed to drain. The drained staples are spread evenly on unbleached kraft paper and air dried using a commercial hair dryer set on cool until the heptane has evaporated. This drying takes approximately 2 minutes. The dried staples are placed in an aluminum dish and spread evenly over the bottom of the dish. The silicone treated staples are cured in a preheated curing oven, after which they are removed from the oven and allowed to cool. A portion of the cooled silicone treated staples are loaded in a surgical stapling instrument.

We have found that solutions containing from 0.6% to 20% and preferably from 3% to 12% by weight of the silicone solids are suitable for treating staples. We have also found that cure temperature of from 100° to 300°C and cure times of from 5 to 60 minutes may be used to cure the silicone treated staples.

For comparative testing, similar degreased stainless steel staples are treated with soap solutions as is well known in the art. Approximately 42.5 g (1-1/2 ounces) of soap is dissolved in 0.45 kg (16 ounces) of water heated to about 90°C. The solution is poured onto the staples loaded in a tray with holes in the bottom of the tray. The staples are allowed to drain, spread on a suitable surface, and blow dried using a commercial hair dryer. A portion of the soap treated staples are loaded in a surgical stapling instrument.

As previously mentioned, silicone has been utilized in many medical devices to prevent tissue ingrowth. However, we have surprisingly found that silicone treated staples, while not only preventing tissue ingrowth, require less force to penetrate the skin, less force to extract the staple from the skin, and unexpectedly the firing of the staple from automated instruments is easier and smoother. The combination of these results produces significantly less trauma in the closing of the wound and in healing the wound. It is extremely difficult to quantitatively measure these properties in vivo. However, the properties may be measured to some degree in vitro; that is, in synthetic materials outside the body. For example, we are able to measure the penetration forces of the staples by the following tests:

Penetration test

One of the held arms of a hemostat is grasped by the upper jaws of an Instron TDDL Testing Machine which has been adapted for compression measurements. The lower jaw of the Instron tester is removed and replaced with a device for holding an approximately 1.27 cm (1/2 inch) thick piece of silicone rubber covered with 50.8 µm (2 mil) thick Mylar® polyester film. The film has a shiny side and a dull side and the dull side is placed against the silicone rubber. An unformed staple having a crown and a pair of legs is carefully bent so that when one leg and approximately 2/3 of the crown is placed in the jaws of the hemostat and the hemostat closed, the other leg points perpendicularly downward to the film covered rubber. The Instron is set at a load of 500 grams, a cross head speed of 1.27 cm (0.5 inch)/minute and a chart speed of 25.4 cm (10 inches)/minute. The hemostat is lowered to the film covered rubber and the staple leg

# 0 092 383

allowed to penetrate the film and the rubber to the full depth of the leg or approximately 3 mm. The force to penetrate the film and rubber is recorded in grams.

The staples are conditioned for one hour prior to testing. Ten staples are tested for penetration force and the mean average penetration force with standard deviation reported.

For comparision purposes, some of the silicone treated staples as made in accordance with Example 1, with the exception that 20% by weight of Syl-Off* is used in the composition, are tested for penetration force. Staples treated with soap as described in Example 1 along with untreated staples are also tested. The Penetration Test as previously described is used. The results of this comparision are given in the following Table I.

TABLE I

| Type of staple | Penetration force (grams) | Standard deviation |
|---|---|---|
| Untreated | 370 | 23 |
| Treated with soap solution | 320 | 16 |
| Treated with Silicone | 203 | 11 |

Example 2

A silicone solution as described in Example is formulated using 20% by weight of Syl-Off* 23—30 in heptane to provide a solution containing approximately 6% by weight of silicone solids. Staples are dipped in the solution, allowed to drain and the solvent evaporated using forced ambient air. Portions of the staples are cured at various conditions and the penetration force of the staples determined. The results of these tests are provided in Table II.

TABLE II

| Cure temperature (°C.) | Cure time (minutes) | Penetration force (grams) | Standard deviation |
|---|---|---|---|
| 110 | 60 | 209 | 13 |
| 250 | 5 | 244 | 17 |
| 250 | 30 | 203 | 11 |
| 250 | 60 | 226 | 18 |
| 300 | 30 | 233 | 16 |
| | uncured | 276 | 18 |

As may be seen from the above data, the cure conditions unexpectedly have little effect on the penetration forces of silicone treated staples.

The force required to extract a staple from embedment in synthetic material may be measured using the following described Extraction Test.

Extraction test

Ten staples from an automatic stapler are fired into neoprene rubber. The rubber is approximately 0.7 cm thick and the staples are fired to span the thickness with the legs penetrating the opposite surfaces of the rubber. The staples are placed about 0.3 cm apart and each staple is clipped on the crown adjacent one of the legs using a pair of metal clipping pliers. The short cut leg of each staple is removed from the rubber. One of the held arms of a hemostat is placed in the upper jaw of an Instron TDDL Testing Machine. A bottom plate replaces the lower jaw of the Instron Tester and the rubber with the embedded staples clamped to the plate so that a leg of a staple is directly beneath the jaws of the hemostat. The jaws of the Instron Tester are brought together and the leg of the staple inserted in the rubber is clamped by the jaws of the hemostat. The Instron Tester is set with a cross head speed of 2.54 cm (one inch)/minute, a load of 200 grams and a chart speed of 25.4 cm (10 inches)/minute. The upper jaw is displaced from the plate holding the rubber and the staple leg removed from the rubber and the force in grams to extract the staple recorded.

The staples are conditioned at 21°C (70°F) and 65% relative humidity for one hour prior to testing. One leg of each of ten staples is tested for extraction force and the mean average extraction force with standard deviation reported.

5

Example 3

Staples treated with soap as described in Example 1 and staples treated at various levels of silicone as described in Example 1 are loaded in surgical stapling instruments. Untreated staples are also loaded in a similar instrument. The soap treated, silicone treated and untreated staples are measured for extraction force using the Extraction Test previously described. The result of the extraction tests are given in Table III.

TABLE III

| Staple type | Extraction force (grams) | Standard deviation |
|---|---|---|
| Untreated | 73 | 12 |
| Soap Treated | 67 | 13 |
| 0.6% by weight Silicone | 56 | 10 |
| 1.8% by weight Silicone | 42 | 5 |
| 2.1% by weight Silicone | 45 | 6 |
| 3.0% by weight Silicone | 44 | 7 |
| 3.6% by weight Silicone | 42 | 10 |
| 4.5% by weight Silicone | 42 | 10 |
| 6.0% by weight Silicone | 30 | 4 |

As may be seen from the above results, even as little as 1.8 percent by weight of silicone in the solution provides the improved extraction results.

Example 4

In order to determine the ease of utilizing silicone treated staples in automated instruments, a blind study is developed. In this study, 10 sterile commercially available disposable staplers are each loaded with 35 silicone treated staples. The staples are treated using a heptane solution containing 1.9% by weight silicone solids. Ten sterile commercially available disposable staplers, exactly the same as the first 10, are each loaded with 35 non-siliconized staples. The second set of staples are treated with soap as described in Example 1. The instruments are tested by free-firing 10 staples; that is, the staple is not fired into solid material or tissue. The next 10 staples are fired into a synthetic stapling material which is neoprene rubber and the next 10 staples are fired into the incised skin of a dog. The staplers are randomized and the staples fired by a surgeon who has no idea which stapling machines have silicone treated staples and which stapling machines do not have silicone treated staples. The results are analyzed. The doctor rates the stapling instruments as to which are the easiest to fire, the next difficult, etc., and the hardest to fire. These ratings are statistically analyzed and the results of the comparisons summarized in Table IV.

6

TABLE IV
Number of staplers with non-silicone treated staples that the stapler with silicone treated staples is better than, worse than, equivalent to.

Test type

| Stapler with silicone treated staples | Free fired | | | Stapling synthetic material | | | Stapling Incised skin | | |
|---|---|---|---|---|---|---|---|---|---|
| | No. better | No. worse | No. equivalent | No. better | No. worse | No. equivalent | No. better | No. worse | No. equivalent |
| 1 | 2 | 3 | 5 | 5 | 1 | 4 | 2 | 1 | 7 |
| 2 | 3 | 2 | 5 | 4 | 2 | 4 | 1 | 1 | 8 |
| 3 | 7 | 1 | 2 | 6 | 1 | 3 | 4 | 0 | 6 |
| 4 | 1 | 2 | 7 | 2 | 3 | 5 | 4 | 0 | 6 |
| 5 | 5 | 3 | 2 | 2 | 1 | 7 | 4 | 0 | 6 |
| 6 | 2 | 4 | 4 | 6 | 3 | 1 | 1 | 2 | 7 |
| 7 | 5 | 1 | 4 | 2 | 2 | 6 | 1 | 0 | 9 |
| 8 | 3 | 1 | 6 | 6 | 0 | 4 | 2 | 0 | 8 |
| 9 | 2 | 4 | 4 | 3 | 1 | 6 | 1 | 0 | 9 |
| 10 | 1 | 0 | 9 | 9 | 0 | 1 | 2 | 0 | 8 |

**0 092 383**

The scores obtained for each test type are further summarized by adding the number under the Number Better Than column to half the number under the Number Equivalent To column. If there was no differences between the silicone treated and non-silicone treated staples, the average score would be 5. The results of these summarizations are presented in Table IV.

TABLE IV
Scores by test type and silicone treated stapler

| Stapler with silicone treated staples | Free fired | Fired into synthetic material | Fired into incised skin |
|---|---|---|---|
| 1 | 4.5 | 7.0 | 5.5 |
| 2 | 5.5 | 6.0 | 5.0 |
| 3 | 8.0 | 7.5 | 7.0 |
| 4 | 4.5 | 4.5 | 7.0 |
| 5 | 6.0 | 5.5 | 7.0 |
| 6 | 4.0 | 6.5 | 4.5 |
| 7 | 7.0 | 5.0 | 5.5 |
| 8 | 6.0 | 8.0 | 6.0 |
| 9 | 4.0 | 6.0 | 5.5 |
| 10 | 5.5 | 9.5 | 6.0 |
| Mean Score | 5.50 | 6.55 | 5.90 |

As may be seen from the above tests, the silicone treated staples have less penetration force required to insert the staple into material, less force required to extract staples from the material and unexpectedly is much easier to fire and, hence, considerably reduces trauma in the closure of and healing of wounds.

## Claims

1. An array of sterile surgical staples for use with a stapling instrument in closing a wound of human or animal skin, comprising a plurality of individual surgical staples, each staple (26) comprising a pair of legs (27, 28) joined together at one end by a crown (29) said staples being disposed in abutting relationship with adjacent staples having their legs and crown touching, characterised in that said staples (26) are adhered together to form an integral array (25) by a polymeric silicone material, the legs (27, 28) of individual staples being coated with a polymeric silicone material whereby it is easier to place said staple in said skin to close a wound and it is easier to remove said staple from said skin when the wound is healed.

2. An array of staples according to Claim 1 wherein the crowns (29) of the staples are coated with a polymeric silicone material to form an integral array (25).

3. An array of staples according to Claim 1 or Claim 2 wherein substantially the entire exposed surface of each staple (26) in the array is coated with a polymeric silicone material.

4. An array according to any one of claims 1 to 3 wherein the silicone material is polydimethyl siloxane.

5. A method of producing an integral array (25) of surgical staples (26) comprising:

aligning a plurality of individual staples (26), each staple comprising a pair of legs (27, 28) joined together at one end by a crown (29), in abutting relationship with adjacent staples having their legs and crown touching and at an angle the same as the angle at which they will be disposed in a surgical stapling instrument, characterised in that:

said aligned staples are treated with a solution containing at least 6% by weight of a polymeric silicone material, and

said treated staples are cured at a temperature of at least 200°C for a period of time of at least 30 minutes to produce an adhered array (25) of staples.

6. The method according to Claim 5 wherein the polymeric silicone material is polydimethyl siloxane.

7. The method according to Claim 5 or Claim 6 wherein the treating solution contains from 9% to 12% by weight of a polymeric silicone material.

8. The method according to any one of claims 5 to 7 wherein the treated staples are cured at a temperature of from 200°C to 300°C for a period of time of from 30 minutes to 60 minutes.

**Patentansprüche**

1. Reihe aus sterilen, chirurgischen Klammern, die mittels eines Klammergeräts zum Verschließen einer Hautwunde von Menschen oder Tieren verwendbar sind, mit mehreren einzelnen chirurgischen Klammern, wobei jede Klammer (26) ein Paar an einem Ende durch eine Krone (29) miteinander verbundene Arme (27, 28) aufweist, wobei die Klammern sich mit ihren Armen und der Krone berührend aneinander anstoßend mit benachbarten Klammern angeordnet sind, dadurch gekennzeichnet, daß die Klammern (26) zum Bilden einer einstückigen Reihe (25) mit einem polymeren Silikonmaterial miteinander verbunden sind, die Arme (27, 28) jeder einzelnen Klammer mit einem polymeren Silikonmaterial beschichtet sind, wodurch es einfacher ist, die Klammer zum Schließen einer Wunde in die Haut einzubringen, und es einfacher ist, die Klammer, nachdem die Wunde verheilt ist, aus der Haut zu entfernen.

2. Klammerreihe nach Anspruch 1, wobei die Kronen (29) der Klammern zum Bilden einer einstückigen Reihe (25) mit einem polymeren Silikonmaterial beschichtet sind.

3. Klammerreihe nach Anspruch 1 oder 2, wobei im wesentlichen die gesamte außenliegende Fläche jeder Klammer (26) in der Reihe mit einem polymeren Silikonmaterial beschichtet ist.

4. Reihe nach einem der Ansprüche 1 bis 3, wobei das Silikonmaterial Polydimethylsiloxan ist.

5. Verfahren zum Herstellen einer einstückigen Reihe (25) von chirurgischen Klammern (26) mit:

Ausrichten mehrerer einzelner Klammern (26), wobei jede Klammer ein Paar an einem Ende durch eine Krone (29) miteinander verbundene Arme (27, 28) aufweist, wobei benachbarte Klammern sich mit ihren Armen und der Krone berührend aneinanderliegen und in einem Winkel, der gleich dem Winkel, in dem sie in einem chirurgischen Klammergerät angeordnet sein werden, ausgerichtet sind, dadurch gekennzeichnet, daß

diese ausgerichteten Klammern mit einer Lösung behandelt werden, die zumindest aus 6 Gewichtsprozent eines polymeren Silikonmaterials besteht, und

die behandelten Klammern bei einer Temperatur von wenigstens 200°C für eine Dauer von wenigstens 30 Minuten ausgehärtet werden, um eine verbundene Reihe (25) von Klammern herzustellen.

6. Verfahren nach Anspruch 5, wobei das polymere Silikonmaterial Polydimethylsiloxan ist.

7. Verfahren nach Anspruch 5 oder 6, wobei die Behandlungslösung 9 bis 12 Gewichtsprozent eines polymeren Silikonmaterials enthält.

8. Verfahren nach einem der Ansprüche 5 bis 7, wobei die behandelten Klammern bei einer Temperatur von 200°C bis 300°C während einer Dauer von 30 bis 60 Minuten ausgehärtet werden.

**Revendications**

1. Un ensemble d'agrafes chirurgicales stériles utilisable avec un instrument d'agrafage lors de la fermeture d'une plate dans la peau d'un être humain ou d'un animal, comprenant une pluralité d'agrafes chirurgicales individuelles, chaque agrafe (26) comportant deux branches (27, 28) jointes ensemble à une extrémité par une âme (29), lesdites agrafes étant disposées côte à côte avec des agrafes adjacentes dont les branches et les âmes se touchent, caractérisé en ce que lesdites agrafes (26) sont collées les unes avec les autres de façon à former un ensemble unitaire (25) à l'aide d'un silicone polymère, les branches (27, 28) d'agrafes individuelles étant revêtues de silicone polymère de façon qu'il soit plus facile de mettre en place ladite agrafe dans ladite peau pour fermer une plaie et qu'il soit plus facile d'enlever ladite agrafe de ladite peau lorsque la plaie est cicatrisée.

2. Un ensemble d'agrafes selon la revendication 1, caractérisé en ce que les âmes (29) des agrafes sont revêtues d'un silicone polymère pour former un ensemble unitaire (25).

3. Un ensemble d'agrafes selon la revendication 1 ou la revendication 2, caractérisé en ce que pratiquement toute la surface exposée de chaque agrafe (26) de l'ensemble est revêtue d'un silicone polymère.

4. Un ensemble selon une quelconque des revendications 1 à 3, caractérisé en ce que le silicone est du polydiméthyl siloxane.

5. Un procédé de fabrication d'un ensemble unitaire (25) d'agrafes chirurgicales (26), consistant à:

— aligner une pluralité d'agrafes individuelles (26), chaque agrafe comprenant deux branches (27, 28) jointes ensemble à une extrémité par une âme (29), placées côte à côte avec des agrafes adjacentes dont les branches et âmes se touchent et orientées d'un angle qui est le même que l'angle selon lequel elles sont disposées dans un instrument d'agrafage chirurgical,

— caractérisé en ce que:

— lesdites agrafes alignées sont traitées avec une solution contenant au moins 6% en poids d'un silicone polymère, et

— lesdites agrafes traitées sont cuites à une température d'au moins 200°C pendant une période de temps d'au moins 30 minutes de façon à produire un ensemble d'agrafes collées (25).

6. Le procédé selon la revendication 5, caractérisé en ce que le silicone polymère est du polydimethyl siloxane.

7. Le procédé selon la revendication 5 ou la revendication 6, caractérisé en ce que la solution de traitement contient de 9 à 12% en poids d'un silicone polymère.

8. Le procédé selon une quelconque des revendications 5 à 7, caractérisé en ce que les agrafes traitées sont cuites à une température comprise entre 200°C et 300°C pour une période de temps comprise entre 30 minutes et 60 minutes.

Fig.1.

Fig.2.

Fig.3.

Fig.6a.

Fig.6b.

Fig.5.

Fig.4.